# EUROPEAN PATENT APPLICATION

(11) **EP 2 216 043 A1**
(43) Date of publication of application: **11.08.2010**
(21) Application number: 08852085.3
(22) Date of filing: 21.11.2008
(51) Int. Cl.: A61K 38/45, A61K 48/00, A61P 43/00

(54) **USE OF INDUCTOR AGENTS GSE24.2 FOR PRODUCING PHARMACEUTICAL COMPOSITIONS FOR TREATING ILLNESSES RELATING TO CELLULAR SENESCENCE**

(30) Priority: 23.11.2007 ES 200703106
(71) Applicant: Consejo Superior De Investigaciones Científicas, 28006 Madrid (ES); Universidad Autónoma de Madrid, 28049 Madrid (ES)
(72) Inventor: PERONA ABELLON, Rosario, E-28029 Madrid (ES); SASTRE GARZON, Leandro, E-28029 Madrid (ES); MACHADO PINILLA, Rosario, E-28029 Madrid (ES); SANCHEZ PEREZ, Isabel, E-28049 Cantoblanco (Madrid) (ES)
(74) Representative: Pons Ariño, Angel
(86) International application number: PCT/ES2008/070212
(87) International publication number: WO 2009/065994

(57) **Abstract**

This invention relates to the use of an inductor or activator compound GSE24.2 for producing a medicament or a pharmaceutical composition for treating a preferably human illness or pathological situation caused by a senescence process. Said pharmaceutical composition can be useful for a treatment for regenerating tissues, for example of epithelial tissues or haematopoietic cells, and also for the immortalisation of eucaryotic cells in order to use of same in biotechnological investigation or processes.

## Description

### SECTOR OF THE TECHNIQUE

Biotechnology sector with human health applications, and more specifically biological compounds - sequences of nucleotides, peptides and human transformed cells - with therapeutic applications for human beings suffering illnesses related to ageing and cell senescence.

### STATE OF THE TECHNIQUE

Telomeres consist of multiple repetitions of the TTAGGG¹ sequence which shorten in each cell cycle due to the inability of DNA polymerases to replicate the end of chromosomes. Maintenance of the telomeres at the end of chromosomes is carried out by the telomerase complex. The protein component of this complex, human telomerase reverse transcriptase (hTERT), contains catalytic reverse transcriptase motifs, whereas the telomere RNA component directs the action of the deoxynucleotide triphosphates (dNTPs) by means of an internal mould complementary to the repeated telomeric sequence TTAGGG ²⁻³. Recently, it has been described how the human telomerase enzyme complex is made up of only two protein components, hTERT and dyskerin, and an RNA component, hTR⁷. Dyskerin is a putative pseudouridine synthase belonging to "H/ACA box" ribonucleoproteins, whereas the H/ACA motif is present in hTR. The complex finally comprises two molecules of each hTERT, hTR and dyskerin.

Telomerase expression is repressed in most somatic cells of adult tissues but remains high in most cancers, contributing to the immortal phenotype in these cells, since it ensures the integrity of the chromosomes.⁶ The levels of telomerase (hTERT) are regulated above all on a transcriptional⁸ level and in humans are controlled by a regulating region in region 5' of the hTERT gene, which is required for maximum activation and contains a binding site for myc (E-box) ⁸⁻⁹. The c-MYC transcription factor stimulates hTERT expression whereas the reinforced expression of mad1 represses hTERT.¹⁰⁻¹¹ Transcriptional regulation of c-myc involves numerous promoters, among which the most important are P1 and P2.¹² The nuclease hypersensitivity element III (NHEIII) of the c-MYC promoter controls 85-90% of c-MYC transcription and was originally identified as the most hypersensitive site to DNAse I¹³. NHEIII contains a purine-rich site on a DNA strand that is capable of establishing a balance between a double helix structure as well as non-B forms in the DNA.¹⁴ The purine-enriched DNA chain can form 2 intramolecular structures of different G quartets¹⁵ which since they act as transcriptional repressors have been used as targets in antitumour therapy.

The shortening of telomeres, or the loss of their secondary structure, causes the cells to go into senescence and/or apoptosis. These two processes act as biological control points which prevent uncontrolled cell division and genetic instability ⁴⁻⁵. Defective regulation of telomere length is involved in the pathology of various illnesses such as premature aging syndromes and cancer.⁶

Senescent cells are viable cells which stop DNA synthesis, flatten and present a cytoplasm rich in vacuoles, having a distinctive gene expression profile and identifiable by means of a biochemical assay that visualises the increased activity of □-gaiactosidase (□-gal) associated to senescence at an acid pH and due to the incapacity of doubling the populations in time.

Senescence in human cells is associated to telomere erosion. Normal non-transformed cells are mortal due to the shortening of the telomeres in each cell division process. At the same time, cancerous cells that express telomerase stabilise the length of the telomeres and become immortal. These findings lead to suggest the hypothesis wherein telomeres act as a biological clock regulating cell ageing.

One of the syndromes of premature ageing associated to telomere erosion is dyskeratosis congenita (DC). This is a very rare, hereditary illness and is characterised by bone marrow failure and a higher propensity to cancer; ¹⁶⁻¹⁸ which are the most important causes of death in these patients. The form linked to the chromosome (X-DC) is caused mainly by sporadic mutations in the DKC gene¹⁹⁻²⁰. This gene encodes dyskerin, a 58 Kd protein containing a pseudouridine synthase domain, which in turn is a component of the telomerase complex. Dyskerin binds the H/ACA boxes present in SnoRNAs (small nucleolar RNAs) and hTR in vertebrates²¹. SnoRNAs function as a mould for dyskerin-mediated RNA pseudouridylation. H/ACA RNAs contain two variable-length ranges, separated by a simple chain region that includes the H box followed by a short sequence containing the trinucleotide ACA ²². There are three different proteins that associate to DKC in the H/ACA of SnoRNAs: Nap2, Gar1 and NOP10 ^{3,22-24}. DKC, Nap2 and NOP10 are intimately associated and are involved in the stability of the ribonucleoproteins complex, whereas interaction with Gar1 is more fluent and transitory^{25, 26-28}. Therefore, dyskerin is required for telomerase activity since it is essential for the accumulation of hTR ^{7,24-29} and the pseudouridylation of ribosomal RNA ³⁰. In patients with DC, the chromosomal instability increases with age as a consequence of the reduction in telomerase activity and the incapacity to maintain the structure of telomeres. In the lymphoblasts and fibroblasts of X-DC patients, telomerase activity and hTR levels are diminished ²¹ and the telomeres are shorter than in unaffected cells. The lack in telomerase activity can be rescued through expression of hTERT and hTR. ^{21,31} Other forms of dyskeratosis congenita occur as a result of hTR mutations or deletions which result in the accelerated shortening of the telomeres and premature death. This defect can only be rescued through re-expression of hTR.³¹

Recently, a genetic suppressor element has been described (GSE) known as 24-2, encoding a pseudouridine synthase domain of the protein dyskerin that allows the telomerase activity to be recovered in the cells of patients with DC-X which has allowed new therapeutic approaches to be developed for this disease and others occurring with alterations of the telomerase complex (ES200502511; SEQUENCE OF NUCLEOTIDES AND PEPTIDES GSE 24.2 OF DYSKERIN, WHICH CAN INDUCE TELOMERASE ACTIVITY, METHOD FOR OBTAINING SAME, THERAPEUTIC COMPOSITIONS AND APPLICATIONS THEREOF). However, knowledge of new activities of this element GSE24.2 would allow new therapeutic and research tools to be developed.

### DESCRIPTION OF THE INVENTION

### Brief Description

One aspect of the invention consists of the use of the inductor or activator compound GSE24.2, hereinafter the use of the invention, for producing a medicament or pharmaceutical composition for treating a preferably human illness or pathological situation, caused by a senescence process.

A particular aspect of the invention consists of the use of the invention wherein the inductor agent GSE24.2 is a sequence of nucleotides, hereinafter gene sequence GSE 24.2 of the invention, which allows expression of a protein or peptide that induces recovery from senescence inside the cells of a mammal, preferably human, and that is made up of one or several sequences of nucleotides GSE 24.2 belonging to the following group:
a) a sequence of nucleotides composed of a human GSE 24.2 nucleotides sequence (SEQ ID N01),
b) a variant of the nucleotide sequence of a) that shows an identity in the sequence of nucleotides of at least 90% with SEQ ID N01,
c) a fragment of any of the sequences of a) and b), and
d) a sequence of nucleotides, genetic construction, which comprises any sequence of a), b) and c).

A more particular aspect of the invention consists of the use of the invention wherein the sequence of nucleotides of the sequence GSE 24.2 of a) is composed of SEQ ID N01.

Another more particular aspect of the invention consists of the use of the invention wherein the sequence of nucleotides of the sequence GSE 24.2 of c) is composed of SEQ ID N03 or SEQ ID NO5, which encode peptide domains Trub I and Trub II, respectively.

Also, another particular aspect of the invention consists of the use of the invention wherein the inductor agent GSE24.2 is a protein or peptide, hereinafter protein GSE 24.2 of the invention, which presents senescence reversal activity inside the cells of a mammal, preferably human, and which comprises one or several sequences of amino acids belonging to the following group:
a) a sequence of amino acids composed of a sequence of human GSE 24.2 amino acids (SEQ ID N02),
b) a variant sequence of amino acids according to the sequence of a) which shows an identity in the amino acid sequence of at least 90% with the polypeptide of SEQ ID N02,
c) a fragment of any of the sequences of a) and b), and
d) a sequence of amino acids that comprises any sequence belonging to a), b) and c).

Another more particular aspect of this invention consists of the use of the invention wherein the inductor agent GSE24.2 is a protein whose sequence of amino acids of a) is composed of SEQ ID N02.

Another more particular aspect of the invention consists of the use of the invention wherein the inductor agent GSE24.2 is a protein whose sequence of amino acids of c) is composed of SEQ ID N04 or SEQ ID N06.

Another aspect of the invention consists of a pharmaceutical composition or medicament for treating an illness or pathology related to senescence alterations, hereinafter pharmaceutical composition of the invention, comprising a compound or activating compound GSE 24.2, in a therapeutically effective amount together with, optionally, one or more adjuvants and/or pharmaceutically acceptable vehicles and that is capable of diminishing or reversing the process of senescence, and obtained by means of the use of the invention.

Another aspect of the invention consists of the use of the pharmaceutical compound of the invention in a procedure for treating a preferably human illness or pathological situation, caused by a process of altered senescence.

Another particular aspect of the invention consists of a eukaryote cell, preferably a human cell, with the senescence process reversed or diminished and comprising element GSE24.2 used in the invention.

### Detailed Description

This invention is based on the fact that the inventors have observed that the expression of protein GSE24-2 (SEQ ID NO1), an isolated fragment of dyskerin (see patent ES200502511), was capable of inducing the recovery of telomerase activity regulating the RNA levels of hTERT and hTR in different types of cells; both in those of DC patients who express the mutated form of dyskerin (patent ES200502511) as well as in those (VA13) with a wild-type allele of DKC and with low telomerase activity (Example 4). In this sense also, in this invention, use of the peptide GSE 24.2 directly (not by means of its gene expression) has been carried out for the first time, demonstrating its capacity to reactivate telomerase activity (Example 1).

At the same time, it has been observed that the protein GSE24-2 activates the transcription of the hTERT protein modulating c-myc expression, since a hybrid myc/mad molecule completely abolishes the transcriptional activation of hTERT. Through an analysis of deletion and mutagenesis it has been observed that the target activator region in the promoter of the human c-myc gene of protein GSE24-2 was the NHEIII domain located beyond region P1. More specifically, it is a polypurine sequence (Pu27) that is also found in other gene promoters such as CCR5 and PDGFA, which is often associated to unusual DNA structures ⁶¹, and that must be kept intact in order to stimulate c-myc transcription (Example 3). Therefore, the alteration of the secondary structure of the G quartets of site NHEIII alters the activity of protein GSE24-2 through this sequence. One must emphasise that there are few known proteins that interact and activate NHE sites meaning that protein GSE24.2 may represent an option for regulating genes whose promoters have said NHE domain. One of them is NM23-H2 or NDP kinase B, a protein that binds to specific DNA sequences and that has an affinity for the NHE site of the c-myc promoter. It also activates the expression of other genes such as myeloperoxidase, CD11b, CCR5 and represses the expression of others such as PDGFA.⁶¹⁻⁶². This protein can also bind in vitro to G-enriched sequences ⁶¹⁻⁶² such as those found by the NHE of the c-myc promoter and telomeres in humans. Therefore, a potential mechanism is one whereby GSE24-2 can activate transcription through NHE by means of its association with proteins such as NM23H2 which facilitate its interaction with DNA and, consequently, produce an increase in the transcription.

The results obtained in X-DC and VA13 cells indicate that the peptide GSE24-2 increases telomerase activity through a combination of two different mechanisms: 1) activating hTERT transcription and 2) increasing hTR levels through stabilisation of this RNA, without it affecting the activity of its promoter. In addition, GSE24.2 could facilitate the assembly of other proteins such as hNaf1 or Nop10 in the H/ACA box, even in the presence of a defective dyskerin.²⁸

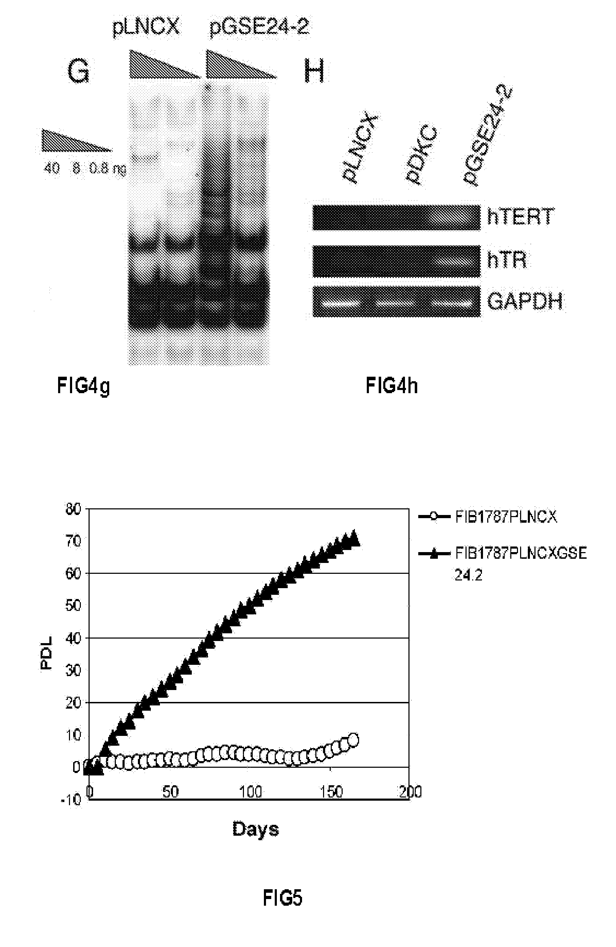

Finally, and surprisingly, it has been observed in this invention that this increase in telomerase activity by means of the GS24.2 protein brings about a recovery from premature senescence of the fibroblasts in X-DC patients in such a way that these cells recover the capacity to multiply, maintaining this activity up to 140 days (Example 4, see Figure 5). This would be mediated by an increase in both hTERT and hTR levels. In this sense, the protein or gene GSE24.2 may constitute a therapeutic approach for illnesses and pathological processes related to cell senescence or constitute a tool for immortalising eukaryote cells or to increase their capacity to proliferate. At the same time, the results of the invention indicate that expression of GSE24.2 does not induce tumorigenicity and would not give rise to aberrant cell growth (Example 3).

In the light of all of the above, GSE24.2 can be used to prepare a medicament or a laboratory reagent useful for treating illnesses or pathological processes related to a process of altered or accelerated cell senescence, including cells that express normal dyskerin such as VA13 cells, or cells that express mutated forms such as autosomal dominant dyskeratosis congenita (DC) or aplastic anaemia.

Therefore, one aspect of the invention consists of the use of the inductor or activator compound GSE24.2, hereinafter the use of the invention, to produce a medicament or pharmaceutical composition for treating a preferably human illness or pathological situation, caused by a senescence process. In this invention, the term "pharmaceutical composition" also includes compositions comprising agent GSE24.2 as described and used in the invention, which are employed in the field of research (as a laboratory reagent) or in industrial biotechnological activity.

As used in the invention, the term inductor or activator compound GSE24.2 refers to a sequence of nucleotides of the GSE 24.2 fragment of dyskerin or a protein or peptide sequence encoding for said sequence of nucleotides that is capable of diminishing or reversing the senescence process inside the cells of a mammal, preferably human. This definition also includes those compounds or molecules that allow expression of a sequence of nucleotides encoding a protein GSE 24.2. An activator compound may be composed of a peptide, a protein or a sequence of nucleotides, an antibody and a polysaccharide.

As used in the invention, the term "illness or pathological situation caused by a normal or accelerated senescence process" refers to an illness in which the cells and tissues present a decrease, whether natural due to aging, or acceleration of their proliferative capacity (see the concept of senescence described in the state of the technique of this invention). Thus, this term as used in the invention refers also to physiological situations altered by the natural ageing of human cells, preferably epithelial or with a high level of proliferation, belonging, by way of illustration without limiting the scope of the invention, to the following group: skin cells, preferably, of the epidermis, gut epithelium, cornea, liver, lung, hair bulb, etc. or cells of the haematopoietic system, preferably, lymphocytes, macrophages and erythrocytes; germinal cells of the testicles and ovaries, etc., respectively, whether somatic cells, stem cells or embryonic cells. Examples of human illnesses or pathological processes in which control of the senescence process and/or telomerase activity can be found, by way of illustration but not limitation, in the revision of Blasco MA (Blasco MA, Telomere length, stem cells and aging. Nature Chemical Biology 3 (10): 640-649, 2007; Crabbe L, Jauch A, Naeger CM, Holtgreve-Grez H., and Karlseder J. Telomere dysfunction as a cause of genomic instability in Werner syndrome. PNAS 104 (7): 2205-2210, 2007).

The reversal or reduction of the cell senescence process can be used therapeutically in those cells with a capacity to divide that maintain organ homeostasis, thereby increasing tissues' capacity to regenerate, irrespective of the cause, whether through ageing, tissue damage from toxins, following surgical interventions, and even in hypoplastic tissues of congenital or hereditary origin, for example, lung hypoplasia. In addition, this reversal or reduction in the cell senescence process can be used to increase the capacity to survive of stem, somatic or embryonic cell populations, in transplant treatments or cell therapy. In these treatments, although stem cells express telomerase, they cannot maintain the length of the telomeres for a prolonged period ⁶ meaning that cell transformation or the treatment with peptide GSE24.2 will increase the functional life of these cells and therefore the efficiency of these cell treatments. Thus, for example, this pharmaceutical composition can be used to regenerate the epidermis in elderly people or can be used to promote skin regeneration in skin wounds or burns, or to stimulate autologous haematopoietic cells that are implanted into a patient undergoing chemotherapy treatment.

Thus, a particular aspect of the invention consists of the use of the invention wherein the inductor agent GSE24.2 is a sequence of nucleotides, hereinafter gene sequence GSE 24.2 of the invention, which allows expression of a protein or peptide that induces the recovery from senescence inside the cells of a mammal, preferably human, and that is composed of one or several sequences of nucleotides GSE 24.2 belonging to the following group:
a) a sequence of nucleotides composed of a human GSE 24.2 nucleotide sequence (SEQ ID N01),
b) a variant of the nucleotide sequence of a) that shows an identity in the sequence of nucleotides of at least 90% with SEQ ID N01,
c) a fragment of any of the sequences of a) and b), and
d) a sequence of nucleotides, genetic construction, which comprises any sequence of a), b) and c).

In the sense used in this description, the term "variant" intends to include any sequence of nucleotides that can be isolated or constructed on the basis of sequence SEQ ID N01, for example, by means of introducing conservative or non-conservative substitutions of nucleotides, including the insertion of one or more nucleotides, the addition of one or more nucleotides to any end of the molecule or the deletion of one or more nucleotides from any end or within the sequence, and that allows encoding a peptide or protein capable of imitating the activity of sequence GSE 24.2 (SEQ ID N02) or fragments thereof (SEQ ID N04 and SEQ ID N06).

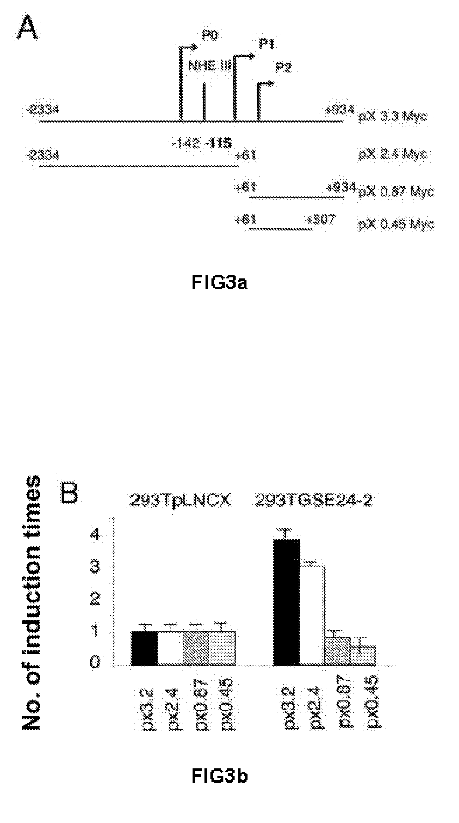

The enzyme dyskerin belongs to a family of pseudouridine synthase that is present in various organisms (see Figure 3B, Mitchel et al, 1999). From the information contained in the invention - and in patent ES200502511 - on different organisms existing in nature a technician skilled in the field of the technique can isolate or construct an analogous sequence of nucleotides to those described herein.

As used in the invention, the term "sequence of nucleotides" refers to a sequence of DNA, cDNA or mRNA.

A more particular aspect of the invention consists of the use of the invention wherein the sequence of nucleotides of sequence GSE 24.2 of a) is composed of SEQ ID N01.

Another more particular aspect of the invention consists of the use of the invention wherein the sequence of nucleotides of the sequence GSE 24.2 of c) is composed of SEQ ID N03 or SEQ ID NO5, which encode the peptide domains Trub I and Trub II, respectively.

The sequence of nucleotides GSE 24.2 identified as d) corresponds to a genetic construction GSE 24.2. This genetic construction GSE 24.2 of the invention can also comprise, if necessary and in order to provide a better isolation, detection or secretion to the cytoplasm of the expressed peptide, a sequence of nucleotides encoding for a peptide likely to be used for purposes of isolation, detection or secretion of said peptide. Therefore, another particular aspect of the invention consists of the use of the invention wherein the sequence of nucleotides is a genetic construction GSE 24.2 that comprises, in addition to the sequence of nucleotides GSE 24.2, any other sequence of nucleotides encoding a peptide or peptide sequence that allows isolation, detection or secretion into the cell cytoplasm of the expressed peptide, for example, by way of illustration without limiting the scope of the invention, a sequence of polyhistidine (6xHis), a peptide sequence recognisable by a monoclonal antibody (for example, for its identification, or any other that serves to purify the resulting fusion protein by means of immunoaffinity chromatography: tag peptides such as c-myc, HA, E-tag) (Using antibodies: a laboratory manual. Ed. Harlow and David Lane (1999). Cold Spring Harbor Laboratory Press. New York. Chapter: Tagging proteins. Pp. 347-377).

The sequence of nucleotides GSE 24.2 and the genetic construction GSE 24.2 described previously may be obtained by an expert by means of well-known techniques in the state of the art (Sambrook et al. "Molecular cloning, a Laboratory Manual 2nd ed., Cold Spring Harbor Laboratory Press, N.Y., 1989 vol 1-3). Said nucleotide sequences may be integrated into a gene expression vector that allows for the regulation of the expression thereof in suitable conditions inside the cells.

Therefore, another particular object of the invention consists of the use of the invention wherein the inductor or activator agent GSE24.2 is an expression vector GSE 24.2 comprising a nucleotide sequence GSE 24.2 or a genetic construction GSE 24.2, described in the invention, and that allows the expression of a protein or peptide capable of diminishing or reversing the process of senescence inside the cells of mammals, preferably humans. A particular mode of embodiment is represented by the expression vector of invention pLNCX 24.2 (see also Spanish patent ES200502511).

In general, an expression vector comprises, in addition to the sequence of nucleotides GSE 24.2 or genetic construction 24.2. described in the invention, a promoter that directs its transcription (for example, pT7, plac, ptrc, ptac, pBAD, ret, etc.), to which it is functionally linked, and other necessary or appropriate sequences that control and regulate said transcription and, where applicable, translation of the product of interest, for example, transcription start and stop signals (tlt2, etc.), polyadenylation signal, replication origin, ribosome binding sequences (RBS), sequences encoding transcriptional regulators, (enhancers), transcriptional silencers, repressors, etc. Examples of appropriate expression vectors can be selected according to the conditions and needs of each specific case among expression plasmids, viral vectors (DNA o RNA), cosmids, artificial chromosomes, etc. that may contain, also, markers that can be used to select cells transfected or transformed with the gene or genes of interest. The choice of vector will depend on the host cell and the type of use required. Therefore, according to a particular mode of embodiment of the invention said vector is a plasmid or a viral vector. Said vector may be obtained using conventional methods known by persons skilled in the art in the same way as different well-known methods can be used for the transformation of microorganisms and eukaryote cells - chemical transformation, electroporation, microinjection, etc. - described in various manuals [Sambrook, J., Fritsch, E.F., and Maniatis, T. (1989). Molecular cloning: a laboratory manual, 2nd ed. Cold Spring Harbor Laboratory, Cold Spring Harbor, N.Y.].

Additionally, another particular aspect of the invention consists of the use of the invention wherein the inductor agent GSE24.2 is a protein or a peptide, hereinafter protein GSE 24.2 of the invention, which presents senescence reversal activity inside the cells of a mammal, preferably human, and which comprises one or several sequences of amino acids belonging to the following group:
a) a sequence of amino acids composed of a sequence of human GSE 24.2 amino acids (SEQ ID N02),
b) a variant sequence of amino acids according to the sequence of a) which shows an identity in the amino acid sequence of at least 90% with the polypeptide of SEQ ID N02,
c) a fragment of any of the sequences of a) and b), and
d) a sequence of amino acids that comprises any sequence belonging to a), b) and c).

As understood in the context of the invention, a variant of a polypeptide as defined in sequence SEQ ID N02 is any peptide that can be obtained from said sequence through substitution, insertion or deletion of one or more amino acids. In the case of variants through substitution, the substitutions are preferably conservative substitutions, in other words, the amino acids are substituted for others having similar characteristics in terms of the properties of their side chain. Thus, conservative substitutions include substitutions within the amino acid groups of table 1.

| **Type of side chain** | **Amino acid** |
|---|---|
| Aliphatic apolar or slightly polar | Ala, Ser, Thr, Pro, Gly |
| Polar with a positive neutral charge | His, Arg, Lys |
| Polar with a negative neutral charge and the corresponding amides | Asp, Asn, Glu, Gln |
| Aromatic | Phe, Tyr, Trp |
| Large aliphatic and apolar | Met, Leu, He, Val, Cys |

Additionally, one or more of the amino acids of the variants of the invention may be substituted by non-conventional natural or synthetic amino acids, such as for example beta-amino acids, 2-aminoadipic acid, alpha-asparagine, 2-aminobutanoic acid, 2-aminocaproic acid, alpha-glutamine, alpha-methylalanine, 2-aminopimelic acid, gamma-amino-beta-hydroxybenzenepentanoic acid, 2-aminosuberic acid, 2-carboxyazetidine, beta-alanine, beta-aspartic acid, 3,6 diaminohexanoic acid, butyric acid, 4-amino 4-amino-3-hydroxybutyric acid, gamma-amino-beta-hydroxycyclohexanepentanoic acid, N5-aminocarbonylornithine, 3-sulfoalanine, 2,4 diaminobutyric acid, diaminopimelic acid, 2,3 diaminopropanoic acid, 2,7 diaminosuberic acid, S-ethylthiocysteine, gamma-glutamic acid, gamma-carboxyglutamic acid, pyroglutamic acid, homoarginine, homocysteine, homohistidine, homoserine, 2-hydroxyisovaleric acid, 2-hydroxypentanoic acid, 5-hidroxylysine, 4-hydroxyproline, 2-carboxyoctahydroindole, 3-carboxyisoquinoline, isovaline, 2-hydroxypropanoic acid, mercaptoacetic acid, mercaptobutyric acid, 4-methyl-3-hydroxyproline, mercaptopropanoic acid, norleucine, nortyrosine, norvaline, ornithine, penicillamine, 2-phenylglycine, 2-carboxypiperidine, sarcosine, 1-amino-1-carboxycyclopentane, statin, 3-thyenylalanine, epsilon-N-trimethyl lysine, 3-thiazolealanine, alpha-amino-2,4-dioxopyrimidine propionic acid.

Additionally, the invention contemplates the use of variants of the peptides of the invention wherein one or more amino acids have undergone changes in their side chain. Examples of side change modifications contemplated in the invention include amino group modifications such as alkylation, amidination, acylation, carbomoylation, trinitrobenzylation, pyridoxylation, modifications of the guanidine group of the arginine residues consisting of the formation of heterocyclic condensates; modifications of the carboxyl groups through amidation, modifications of tyrosines through methoxylation, modification of the imidazole ring of histidine through alkylation or N-carboxyethylation, modifications of proline through hydroxylation in position 4. Alternatively, the invention contemplates variants of the peptides of the invention through glycosylation, in other words, the addition of glycan groups either to the side chain serine and/or threonine (O-glycosylation) or to the side chain asparagine and/or glutamine (N-glycosylation). The glycans that can be incorporated into the polypeptides of the invention include a variable number of glucidic units (mono-, di-, tri, tetrasaccharides and so on). The monosaccharides that form in glycan include D-allose, D-altrose, D-glucose, D-mannose, D-gulose, D-idose, D-galactose, D-talose, D-galactosamine, D-glucosamine, D-N-acetylgucosamine, D-N-acetylgalactosamine, D-fucose or D-arabinose.

Alternatively, the invention contemplates the use of variants of the polypeptides described in the invention wherein the D stereoisomers are included of at least one of the amino acids constituting the peptide chain in order to give rise to the retro-inverse isomers.

In another mode of embodiment, the invention contemplates the use of peptidomimetics of the polypeptides described in the invention, in other words, variants wherein one or more of the peptide bonds have been replaced by an alternative type of covalent bond. Said peptidomimetics are **characterised in that** they show greater stability as they are more resistant to proteases. Modifications of the peptide skeleton include substitution or insertion in elements of the peptide bond (-NH-, -CH-, -CO-) of groups such as -O-, -S-, - CH2 instead of -NH-, -N-, -C-alkyl p -BH- instead of -CHR and -CS-, -CH2-, - SOn-, -P=O(OH)- or -B(OH)- instead of -CO-. In addition, it is possible to increase the stability of the peptides of the invention using groups that block the N-terminal end such as t-butyloxycarbonyl, acetyl, succinyl, methoxysuccinyl, suberyl, adipyl, dansyl, benzyloxycarbonyl, fluorenylmethoxycarbonyl, methoxyadipyl, methoxyadipyl, methoxysuberyl 2,3-dinitrophenil. Alternatively or simultaneously, it is possible to modify the C-terminal end of the peptides through amidation.

Determination of the level of identity between the variants and the polypeptides defined in sequence SEQ ID N02 is carried out using computer algorithms and methods well-known by a person skilled in the art. Preferably, the identity between two sequences of amino acids is determined using the BLASTP algorithm (BLASTManual, Altschul, S., et al, NCBI NLM NIH Bethesda, Md. 20894, Altschul, S., et al., J. Mol. Biol. 21 5: 403-410 (1990). Preferably, the polypeptides used in the invention show a sequence identity with the polypeptides defined in the sequence of SEQ ID NO: 1 to 19 of at least 50%, at least 60%, at least 70%, at least 80%, at least 90%, at least 91%, at least 92%, at least 93%, at least 94%, at least 95%, at least 96%, at least 97%, at least 98% or at least 99%.

The enzyme dyskerin belongs to a pseudouridine synthase family that is present in various organisms (see Figure 3B, Mitchel et al, 1999). From the information described in this invention and different organisms existing in nature an expert technician in the field of the technique can isolate or construct a sequence of amino acids similar to those described in the invention.

Another more particular aspect of the invention consists of the use of the invention wherein the inductor agent GSE24.2 is a protein whose amino acid sequence of a) is composed of SEQ ID N02.

Another more particular aspect of the invention consists of the use of the invention wherein the inductor agent GSE24.2 is a protein whose amino acid sequence of c) is composed of SEQ ID N04 or SEQ ID N06.

On a separate note, another additional aspect of the invention consists of cells, whether eukaryote - preferably human, more preferably isolated - or prokaryotes, hereinafter GSE 24.2 cells of the invention, genetically modified and comprising the sequence of nucleotides, the construction and the expression vector GSE 24.2 of the invention and wherein the peptide or protein GSE 24.2 used in the invention can be suitably expressed. These cells can be transformed, infected or transfected by means of said nucleotide sequences using genetic engineering techniques known to persons skilled in the art [Sambrook, J., Fritsch, E.F., and Maniatis, T. (1989). Molecular cloning: a laboratory manual, 2nd ed. Cold Spring Harbor Laboratory and form part of the invention. These cells can be useful for producing peptides capable of reversing or diminishing the senescence process and that can serve as the basis for a pharmaceutical composition, for the recombinant amplification of said nucleotide sequences, or can be useful per se as cells in gene therapy, etc. A particular embodiment would be the use of human cells transformed by means of these GSE 24.2 nucleotide sequences, from different cell lineages, which can be used as human tissue regenerator cells.

In another aspect, the invention relates to a cell or host organism that contains a genetic construction of the invention or a vector as defined in the invention. Any type of host organism known to a person skilled in the art can be used in the invention, such as a bacteria strain (Escherichia coli, Bacillus subtilis and similar), a yeast strain (Saccharomyces cerevisiae, Pichia pastoris, Kluyveromyces lactis, Hansenula polymorpha and similar), a transgenic plant (dicotyledoneae or monocotyledoneae), the cell of an insect, for example, baculovirus, the cell of a mammal (cells COS, CHO, C127, HeLa and similar) and a non-human transgenic (for example, a mouse, a cow, a goat, a rabbit, a pig etc.).

Gene expression systems may or may not allow integration of the new genetic material into the genome of the host cell. In this way, both the sequence of nucleotides, genetic construction or the expression vector GSE 24.2 can be used as a medicament to protect host cells, preferably human cells affected by a process of senescence, altered (increased) or not, in a treatment and prevention procedure of gene therapy on a human being affected by an illness related to an altered senescence process. Similarly, cells GSE 24.2 of the invention can be used as a medicament for the regeneration or implantation of tissues or cells in human beings. The biopharmaceutical tools and gene therapy procedures are sufficiently known by a person skilled in the field of the technique for the information described in the invention to be developed without excessive effort. In addition, the proteins or peptides and the cells themselves can become biodrugs.

Another aspect of the invention consists of a pharmaceutical composition or medicament for treating an illness or pathology related to alterations of senescence, hereinafter pharmaceutical composition of the invention, comprising a compound or activator compound GSE 24.2, in a therapeutically effective quantity together with, optionally, one or more adjuvants and/or pharmaceutically acceptable vehicles and that is capable of diminishing or reversing the process of senescence, and obtained by means of use of the invention.

For use in medicine, the compounds and combinations of compounds of the invention can be formulated jointly with an excipient that is acceptable from a pharmaceutical point of view. Preferred excipients for their use in the invention include sugars, starches, celluloses, gums and proteins.

In the sense used in this description, the term "therapeutically effective amount" refers to the quantity of the agent or compound capable of recovering from senescence, calculated in order to produce the desired effect and, will be determined, in general, among other causes, by the inherent properties of the compounds, including the age, state of the patient, severity of the alteration or disorder, and route and frequency of administration.

In a particular embodiment, the pharmaceutical composition of the invention will be formulated in a pharmaceutical form for solid administration (e.g., tablets, capsules, lozenges, granules, suppositories, etc.) or liquid (e.g., solutions, suspensions, emulsions, etc.). In another particular embodiment, the pharmaceutical compositions of the invention may be administered via any route, including, without limitation, oral, intravenous, intramuscular, intraarterial, intramedullary, intratecal, intraventricular, transdermic, subcutaneous, intraperitoneal, intranasal, enteric, topical, sublingual or rectal. A review of the various forms of administration of active principles, the excipients to be used and their manufacturing procedures can be found in the Galenic Pharmacy Treatise (Tratado de Farmacia Galénica), C. Faulí i Trillo, Luzán 5, S.A. de Ediciones, 1993.

Another aspect of the invention consists of the use of the pharmaceutical composition of the invention in a procedure for treating an illness or pathological situation, preferably human, caused by a process of altered senescence.

No less important, another particular aspect consists of the use of the pharmaceutical composition of the invention in a procedure for immortalising eukaryote cells, preferably of humans or of animals used in biomedical research. The immortalisation of eukaryote cells is a highly necessary practice in the field of biotechnological R + D since it increases the useful life thereof, in relation to recombinant protein production processes, preferably with biomedical applications, or of normal cells that are used to analyse the effects of potential therapeutic compounds and where longer periods of time would be convenient in order to analyse secondary effects over the long term.

Another particular aspect of the invention consists of a eukaryote cell, preferably a human cell, with the senescence process reversed or diminished and that comprises the element GSE24.2 used in the invention.

### DESCRIPTION OF THE DRAWINGS

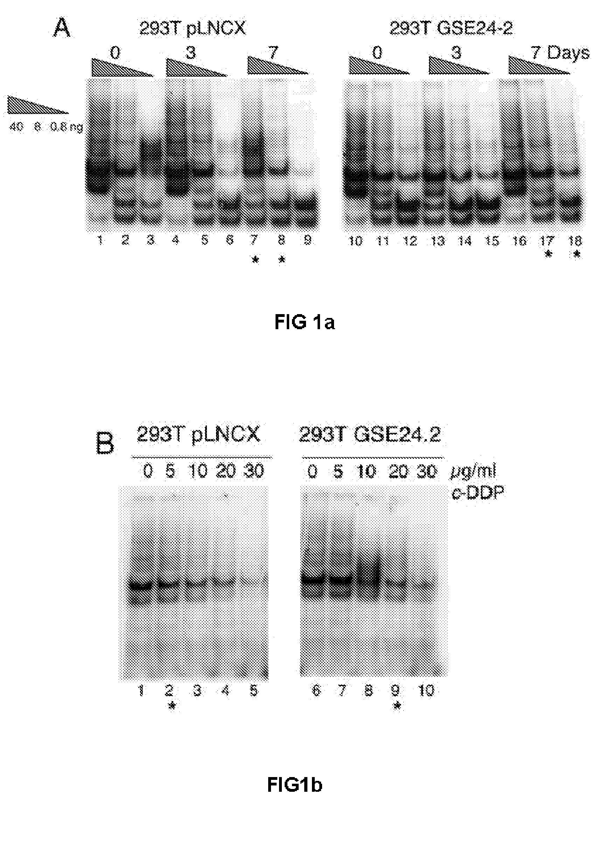

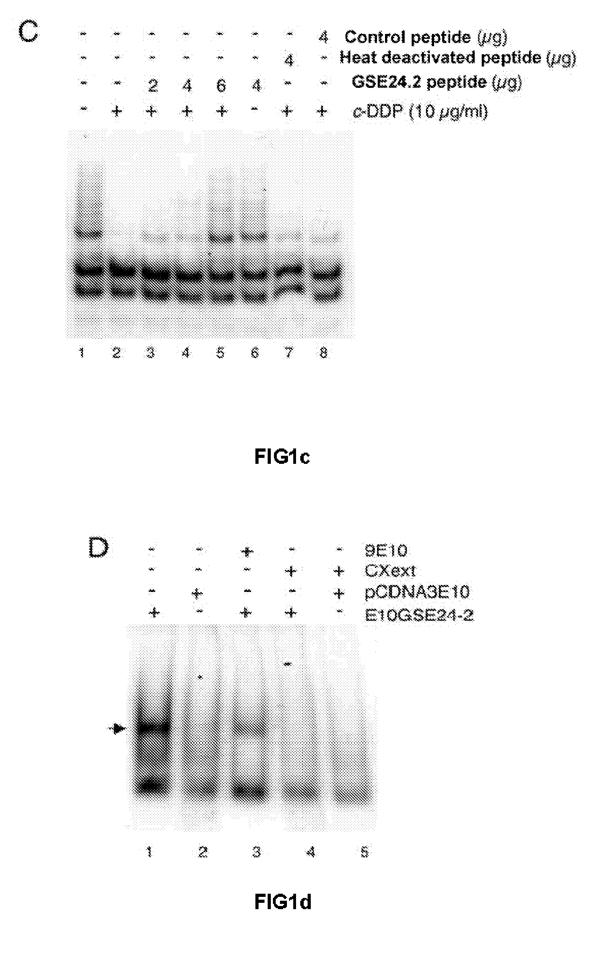

**Figure 1****.- Effect of the expression of peptide GSE24-2 on telomerase activity in response to cisplatinum. (C)** Telomerase activity following in vitro treatment of 293T cell extracts with cisplatinum and peptide GSE24-2. After treating 293T cell extracts in vitro with cisplatinum and rising amounts of purified peptide GSE24-2 (lines 3-5), heat-deactivated peptide GSE24-2 (line 7) or a control peptide (line 8) the latter were used in a TRAP assay. In order to carry out the telomerase assay, a specific mould was used with the telomeric sequence. The experiment was repeated 3 times with similar results. (D) Gel shift tests using cell extracts that expressed GSE24-2. The nuclear extracts of cells expressing plasmid pcDNA3-9E1024-2 or pcDNA3-9E10 were subjected to a gel shift test. A TEL1 probe was used containing the specific telomeric sequence. The specificity of the bond was established by using a specific antibody 9E10 (line 3) and oligonucleotide CXext, which hybridised to TEL1, acts as a competitor (lines 4-5). The experiment was repeated three times with similar results.

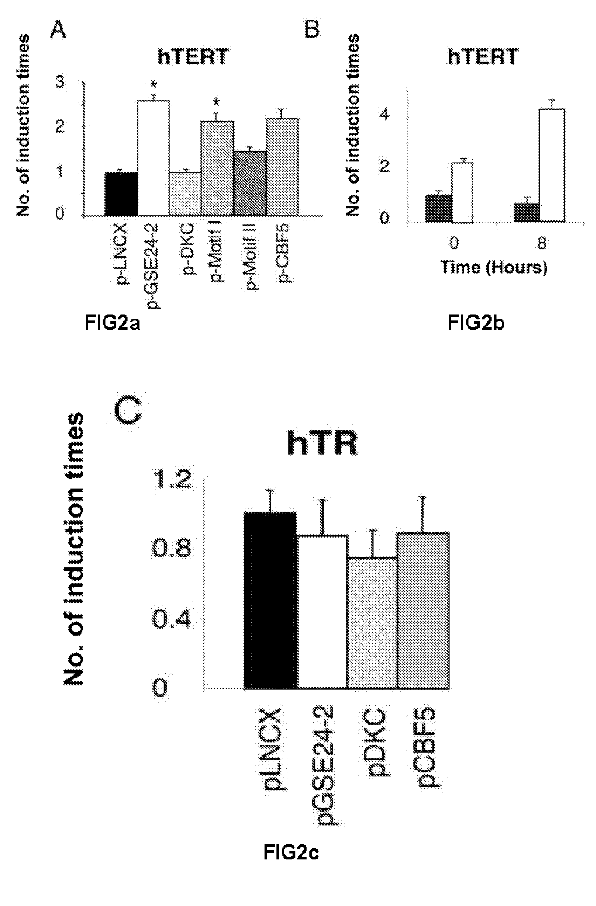

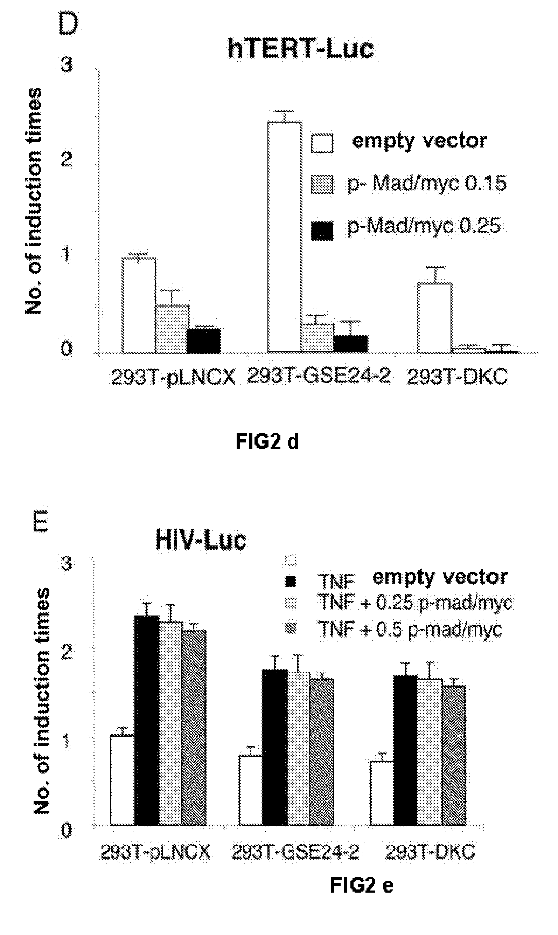

**Figure 2****.- GSE24-2 increases the activity of the hTERT promoter. (A)** The 293T cells were co-transfected with pLNCX, DKC, GSE24-2, Cbf5, motif I or motif II (10 µg DNA per million cells) and the reporter plasmid hTERT-luc (1 µg per million cells). Telomerase activity was measured 24 h after transfection. *Indicates values with a statistical meaning of p<0.05. **(B)** The cells 293T (black) and 293T24-2 (white) were co-transfected with the reporter vector hTERT-luc (1 µg per million cells). After 24 hours, the cells were treated with cisplatinum (3 □g/ml) during 8 h and luciferase activity was measured. **(C)** The 293T cells were co-transfected with the indicated constructions (10 µg DNA per million cells) and the reporter vector hTR-luc (1 µg per million cells). Luciferase activity was measured 24 hours after transfection. **(D)** The cell lines were co-transfected with the reporter vector hTERT-luc and different amounts of the expression vector for Mad/myc. Luciferase activity was measured 24 hours after transfection. **(E)** The cell lines were co-transfected with the reporter vector HIVluc and rising amounts of the expression vector mad/myc. 24 hours after transfection they were stimulated with 50 ng/ml of TNF□ during 6 hours and luciferase activity was measured. As a transfection efficiency control, the CMV-Renilla vector was used (0.1 µg/ml per million cells). Each point represents the mean and standard deviation of two experiments carried out in quadruplicate.

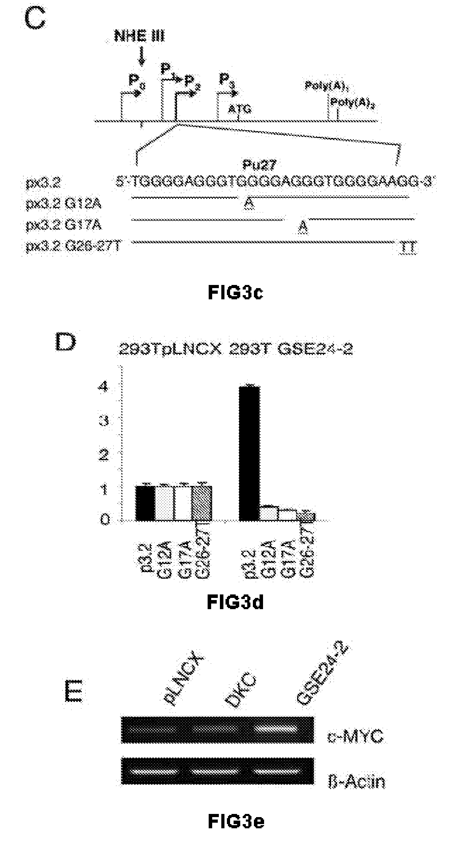

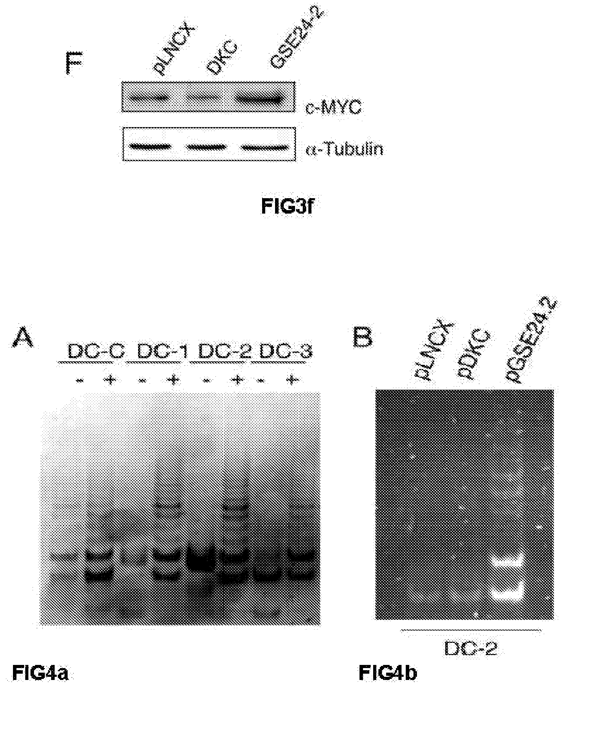

**Figure 3****.- GSE24.2 induces its activity through the promoter of the c-MYC gene. (A)** Schematic outline of the c-MYC promoter indicating the different constructions used in the experiments. **(B)** The cells 293T pLNCX and 293T GSE24-2 were transfected with different constructions of the reporter vector c-MYC-luc (1 µg per million cells). Luciferase activity was measured 24 hours after transfection. As a transfection efficiency control, the CMV-Renilla vector was used (0.1 µg/ml per million cells). Each point represents the mean and standard deviation of two experiments carried out in quadruplicate. **(C)** Schematic outline of the mutations generated in NHE III. **(D)** The indicated cell lines were transfected with the mutant reporter vectors derived from plasmid px3.2 (1 µg per million cells). Luciferase activity was measured 24 hours after transfection. As a transfection efficiency control, the CMV-Renilla vector was used (0.1 µg/ml per million cells). Each point represents the mean and standard deviation of two experiments carried out in quadruplicate. **(E)** The MEF cells were transfected with the expression vectors pLNCX, DKC or GSE24-2. 24 hours after transfection total RNA was isolated and expression of the RNA of the mouse gene c-myc and □-actine was measured by means of RT-PCR. The experiment was repeated three times with similar results. **(F)** The MEF cells were transfected with the expression vectors pLNCX, DKC or GSE24-2. 24 hours after transfection total protein extracts were isolated, and expression of the mouse protein c-myc and □tũbulin was determined by means of a western blot using specific antibodies. The experiments were repeated three times with similar results.

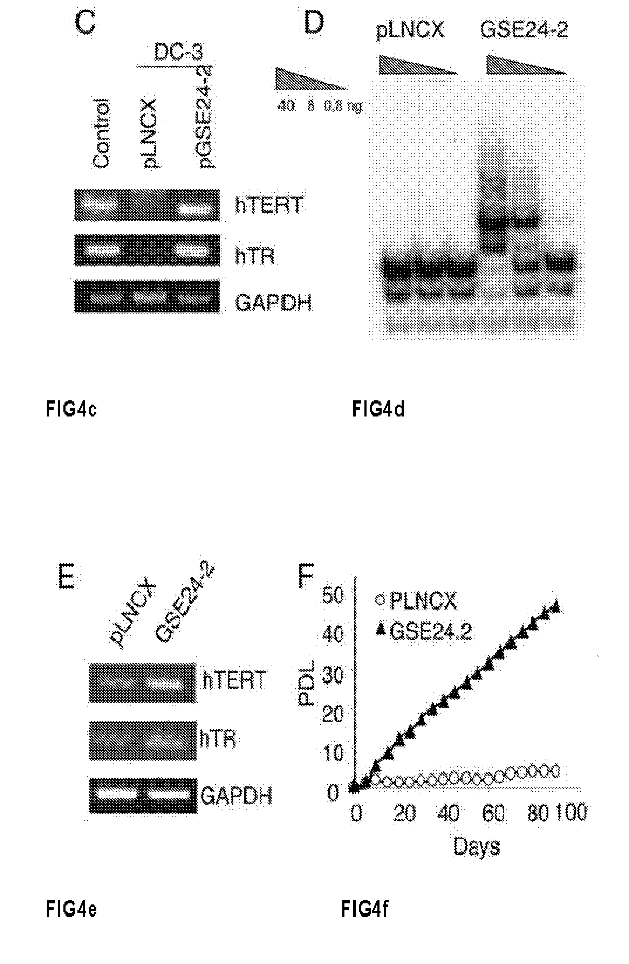

**Figure 4****.- GSE24-2 reactivates telomerase activity in the cells of patients with dyskeratosis congenita associated to the X chromosome and in VA13 cells. (A)** Telomerase activity was measured in the lymphoblasts of patients suffering from dyskeratosis congenita associated to the X chromosome obtained taken from a carrier mother (DC-C) and affected offspring (DC-1, DC-2 and DC-3). The cells were transfected transiently with 3 µg of empty vector (-) or GSE24-2 (+) per million cells. Telomerase activity was measured 24 hours later. **(B)** Cells DC2 were transfected transiently with 3 µg of expression vectors pLNCX, GSE24-2 or DKC, per million cells. Telomerase activity was measured 24 hours later. **(C)** The levels of expression of hTERT and hTR in the cells of one of the patients (DC-3) were transfected with 3 µg of empty vector or GSE24.2 per million cells. RNA levels were detected by RT-PCR. GAPDH expression was used as a control. **(D)** The fibroblasts from patients with X-DC GMO1787 were infected with the viruses derived from vectors pLNCX or GSE24-2 and the stable cell lines were used to determine telomerase activity (the total amount of protein is shown in the triangle). (E) The expression levels of hTERT and hTR in GMO1787 cells transfected with plasmids pLNCX and pGSE24-2 were determined by RT-PCR. GAPDH expression was used as a control. **(F).** The speed of growth of GMO1787 cells infected as described in panel D has been estimated by means of the accumulation of duplications of the population in time. All experiments have been repeated three times, with similar results. **(G)** Telomerase activity in VA13 cells. The cells were transfected transiently with 16 µg of the control vector pLNCX or GSE 24.2 per million cells. Telomerase activity was measured 24 hours later using seriated dilutions of protein extracts (The total amount of protein appears in the triangles). **(H)** Expression levels of hTERT and hTR in VA13 cells transfected with 16 µg of the plasmids pLNCX, DKC or GSE24-2/per million cells. RNA levels were determined by RT-PCR. GAPDH levels were used as a control.

**Figure 5****.- Peptide GSE24.2 recovers from cell senescence.** The speed of growth of GMO1787 cells infected as described in panel 4D has been estimated by means of the accumulation of population duplications in time. All experiments have been repeated three times with similar results.

### EXAMPLES OF THE INVENTION

### Example 1.- Peptide GSE 24.2 rescues telomerase activity in vitro

The telomere sequence TTAGGG is a target for cisplatinum ⁴⁵. The result of the cisplatinum bond to this region brings about the inhibition of telomerase and the shortening of telomeres ⁴³. For this very reason, since dyskerin is one of the components of the telomerase complex ⁷ we proceeded to study the effect of cisplatinum and the expression of GSE24-2 in telomerase activity by means of a TRAP ³⁷ assay, observing that cells transfected with a vector that allows the expression GSE24-2 presented higher telomerase activity (Patent ES200502511).

This description for the first time describes use of the previously described peptide GSE 24.2 in an *in vitro* test of telomerase activity inhibition using cisplatinum with cell extracts from 293T cells and a telomere probe treated with cisplatinum. In this test, the peptide GSE24-2 was added in a dose-dependent manner, in order to protect the telomerase activity of the extracts (Figure 1C, lines 3-5) from the inhibition by cisplatinum. At the same time, heat deactivated peptide GSE24-2 or an unrelated peptide were incapable of rescuing the inhibition of telomerase induced by cisplatinum. (Fig 1C, lines 7-8), indicating that peptide GSE24-2 was capable of inhibiting the crosslinking induced by cisplatinum in telomeres *in vitro.* Additionally, the possible bond of the peptide GSE24-2 to the telomere sequence was analysed using gel shift tests, with an oligonucleotide that forms a loop (TEL1) which reconstructs the double chain sequence of the telomere with a small 3' hanging sequence ⁴¹. A specific complex GSE24-2/TEL1 (Fig 1D) was detected. The oligonucleotide Cxext, which hybridises with the simple chain extension 3', completely inhibits the formation of the complex GSE24-2/TEL1. In conjunction, these results indicate that GSE24-2 is capable of binding to telomeric sequences and that the lack of inhibition of telomerase activity by cisplatinum may be in part a protective effect of GSE24-2 on the telomeric probe.

### Example 2.- The promoter of hTERT but not of hTR is activated by protein GSE24-2.

Previously it was observed that the capacity of GSE24.2 to protect against cisplatinum was due to its capacity to maintain high RNA levels of hTERT (ES200502511). According to this hypothesis, 293T cells that overexpress hTERT were more resistant to cisplatinum than the control cells.

Given that cisplatinum induces a reduction in the mRNA of hTERT and the expression of GSE24-2 appears to compensate this effect, we investigated whether this could be due to a change in the transcription of hTERT.⁵³ We researched the activity of the hTERT promoter in cells GSE24-2 using a reporter vector containing 3402 pb of the promoter.³³ The expression of GSE24-2, but not of DKC, was capable of stimulating the transcription of the hTERT promoter in transient transfection tests (Figure 2A). Also, cisplatinum stimulates the promoter's activity in GSE24-2 cells more than in the control cells (Figure 2B). These results indicate that the expression of GSE24-2 was capable of stimulating the transcription even in the presence of cisplatinum, resulting in increased levels of hTERT mRNA. We have also been capable of detecting stimulation of the hTERT promoter in MEF cells (primary mice fibroblasts) transfected with the plasmid GSE24-2 (data not shown). No effect was observed of the expression of GSE24-2 on the promoter hTR ⁹ using cells expressing DKC or GSE24-2 (Figure 2C). However, the treatment with inhibitor JNK SP60125⁹ was capable of activating the activity of this promoter (data not shown) as described previously ¹¹. These results indicate that the activity of GSE24-2 is specific for hTERT expression.

Dyskerin was originally identified as a homologous human gene of cbf5 ⁵⁵ which shows 85% identity with the Cbf5 gene of *S. cerevisiae* on a protein level ⁵⁶. Therefore, we cloned from the yeast gene *S. cerevisiae* cbf5 the sequence equivalent to GSE24-2 (SEQ ID N02) in the vector pLNCX. The expression of this construction also increases the expression of the hTERT promoter (Figure 2A), but not of the hTR promoter (Figure 2C). This indicates a high degree of conservation in the function of this region of DKC.

### Example 3.- The NHEIII domain of the c-MYC promoter is the target sequence of GSE 24-2.

The hTERT promoter contains two E-boxes which can bind myc/max heterodimers ^{32,57}. A hybrid molecule that contains the c-myc DNA-binding domain, the one that transactivates mad, is capable of binding to the E boxes, and therefore inhibits c-myc dependent transcription. The expression of mad/myc was capable of inhibiting the activity of the hTERT promoter in a manner that was dependent on the doses in the control cells as well as in the DKC cells (Figure 3D). Also, the expression of myc/mad was capable of abolishing the transcriptional activation mediated by the protein GSE 24.2 in cells GSE24-2. This indicates that the activation of the hTERT transcription induced by GSE24-2 is c-myc-dependent (Figure 3D). The inhibition is specific since the transfection of the myc/mad construction together with a reporter vector for the NFκB promoter (HIVLuc) did not affect the transcription induced by TNFα (Figure 4E).

A reporter vector containing 3.2 Kb of the c-myc gene (px3.2myc), and including promoters P0, P1 and P3 bound to the luciferase gene (Figure 3A) was transfected in control cells and GSE24-2 cells. An increase of three times the promoter's activity in GSE24-2 cells in relation to the control cells was observed (Figure 3B). A similar result was obtained when 293T cells were transfected with the vector pLNCX or a plasmid expressing GSE24-2 together with reporter vector px3.2myc (data not shown). Three deletion mutants of the c-myc promoter were used to define the region necessary for GSE24-2-mediated activation (Figure 3A). Only those constructions that contained distal promoters P1 and P0, which included NHEIII, were capable of being activated in GSE24-2 cells, but not in control cells (Figure 3B). This indicates that the proximal promoter P2 is not involved in GSE-mediated activation.

Different mutants of NHEIII were obtained by means of substituting guanine residues in the purine-rich region involved in maintaining the secondary structure of the G-quartet (Pu27). Mutants of G12A were obtained in the second G group of the quartet, G17A from the second triplet of G and finally two consecutive guanines (G26A/G27A) at the end of the purine-rich region (Figure 3C). The results indicate that only the original sequence is activated by GSE24-2 (Figure 3D). As a whole these results suggest that GSE24-2 is capable of inducing a modification of the secondary structure of the Pu27 region in an active arrangement that allows c-myc transcription.

We also studied whether the changes observed in the activity of the c-myc promoter were reflected in the expression thereof. To this effect, MEF cells were transfected transiently with empty, DKC or GSE24-2 expression vectors and next mRNA and c-myc protein levels were measured (Figure 3E y 3F). As expected, only the expression of GSE24-2 was capable of inducing an activation of the expression, both of the protein as well as of the c-myc mRNA. One of the unwanted effects of GSE24-2 expression could be that the increase in c-MYC expression could induce cell transformation. In order to evaluate this possibility, atimic mice were inoculated with Pam212 cells, expressing empty vector, human c-myc gene, or GSE24-2. Only the animals inoculated with cells expressing c-myc developed tumours within the first 10 days whereas the cells expressing GSE24-2 did not developed tumours even three months after inoculation.

### Example 4.- Telomerase activity in VA13 cells and X-DC patients, stimulated using GSE24-2 recovers them from the senescence process.

We have studied the effect of GSE24-2 expression in cells of patients suffering from X-DC. Commercial lymphoblasts were used taken from a carrier mother (DC, normal dkc allele) and her three clinically affected offspring (DC1, DC2 and DC3, mutant dkc allele). These cells are not immortalised and normally in culture go into senescence. On top of what was previously observed, in other words the expression of GSE24-2 increases telomerase activity on all these cell lines (Figure 4A), an increase in telomerase activity with full dyskerin expression was not observed (Figure 4B) as described previously ²¹. The levels of RNA of hTERT and hTR are increased in cells transfected with GSE24-2 (Figure 5C) suggesting that recovery of telomerase activity with transfection of GSE24-2 is due to the increase in both molecules.

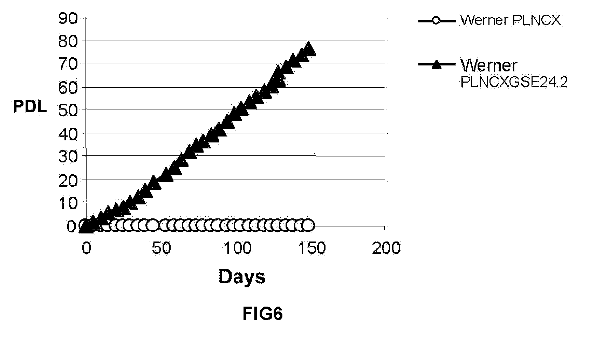

At the same time, the primary dermal fibroblasts of X-DC patients become totally senescent within 3-4 weeks of continuous culture, whereas the normal cells proliferate until two months before reaching the senescence phase ²¹. The X-DC cells that express hTERT acquire the capacity to divide for longer and grow continuously for several months ²¹. The expression of GSE24-2 in GMO1787 cells of X-DC, as already seen previously, was capable of inducing an increase in telomerase activity (Figure 4D) by means of an increase in the expression of RNA levels of hTR and hTERT in fibroblasts of X-DC (Figure 4E), and, surprisingly, for these cells to be capable of growing continuously (Figure 5) up to 160 days. The cells (senescent dermal fibroblasts) of patients with Werner's syndrome were also capable of growing continuously (Figure 6) up to 150 days.

More interestingly, in the VA13 telomerase-deficient cell line (wild-type dyskerin) which presents a lack of hTERT and hTR ⁵⁹ and maintains telomeres by means of an ALT mechanism, the expression of GSE24-2 was capable of recovering telomerase activity (Figure 4G), and RNA levels of hTERT and hTR (Figure 4H).

### Materials and methods

**Constructions and cell lines.** The sequences GSE24-2, DKC, DKC5', motif I, motif II and the region of the CBF5 gene homologous to GSE 24-2 were cloned in the vector pLNCX (BD Biosciences, Madrid). The construction with the hTERT promoter was obtained from Dr. T.Kim³², the construction with the hTR promoter was obtained from Dr. N.Keith ⁹, the ones with the c-MYC promoter (px3.2) from Dra. A. Aranda and the hybrid construction Myc/mad, pECL and pECLc-myc from Dr. J. León. The plasmid BABE-TERT was obtained from Dra. K. Collins ³¹. The plasmid PGATEV ³³ was obtained from Dr. G. Montoya. The plasmid PGATEV-24-2 was obtained by subcloning the 24-2 fragment in the Ndel/Xhol sites of the pGATEV plasmid. Plasmid pCDNA3-9E10-24-2 was obtained by subcloning the 24-4 fragment between the EcoR1/Xbal sites of vector pCDNA3 containing epitope 9E10-myc. The 293T cells (American Type Culture Collection) Phoenix were cultured in DMEM medium supplemented with 10% FBS. The cell line VA13 was obtained from Dr. M. Serrano. The X-DC cells were obtained from the Coriell Cell Repository and were cultivated in RPMI supplemented with 20% FBS. The X-DC lymphoblasts of the family (DC, DC1, DC2 and DC3) have been clinically described ³⁴⁻³⁵ and the dyskerin gene presents one single substitution: T66A. The X-DC skin fibroblasts GMO1787 were cultured in DMEM supplemented with 10% FCS and encoded by a dyskerin protein lacking leucine 37 ³¹. Murine embryonic fibroblasts (MEF) were cultured in DMEM supplemented with 10% FBS and non-essential amino acids (Gibco, Carlsbad, USA). For stable transfections the 293T cells were co-transfected with 10 µg/million cells of GSE24-2 or pLNCX and 1 µg of the plasmid p-BabePUR (Clontech) and selected for puromycin-resistance 24 h after transfection. The Pam 212 cells were transfected with the vectors pECL, pECLc-myc or GSE24-2 and selected for resistance to G418. For the infection of X-DC fibroblasts, the Phoenix cells were transfected with the vectors derived from pLNCX using the calcium phosphate method. 48 hours after transfection the virus were collected and used to infect X-DC fibroblasts during 48 hours. The stable cell lines were selected with G-418 (Gibco).

**Library of GSEs and selection with cisplatinum.** The library of GSEs was constructed essentially as described previously by Roninson et al³⁶ **Reagents.** The cisplatinum, inhibitor of Telomerase I and SP60125 were purchased from Calbiochem (San Diego, USA). The TNFα was purchased from Upstate (Charlottesville, USA).

**Production and purification of the peptide GSE24-2:** The DH5α cells of E. coli were transformed with the vector pGATEV GSE24-2 and the lysates were prepared as described previously. ³³. The fusion protein was purified with glutathione-sepharose and the purity was analysed by means of gel electrophoresis. The GSE24-2 was obtained following digestion with the protease TEV following the manufacturer's instructions. Routinely, 90% was obtained of the digested protein according to what was obtained in SDS-PAGE electrophoresis. The protein was passed twice through a Hi-Trap Ni-NTA column in order to discard polyhistidine tails, undigested protein, TEV protease, and impurities.

**Directed mutagenesis.** The mutations of guanine residues of the NHEIII region of the c-MYC promoter (px3.2) were carried out using the Quickchange X-L site-directed mutagenesis kit (Stratagene, Santa Clara, USA) following the manufacturer's instructions.

**Telomere repeat amplification protocol (TRAP).** Telomerase activity was measured using the TRAPeze³⁷ telomerase detection kit (Intergen, Purchase, USA) following the manufacturer's instructions. The TRAP assay was carried out labelling each protein extract by total present protein concentration. In the indicated experiments, a telomeric probe was used in the TRAP assay ³⁸. *In vitro* platination was carried out by incubating with cisplatinum directly in the TRAP reaction.

**Immunoblots and antibodies.** Cells were lysed as described previously ³⁹ and twenty µg of protein were analysed in SDS-PAGE gels at 10%. The antibodies employed were: anti-pJNK (V7391, Promega, Madison, USA), anti-JNK1 (C-17, Santa Cruz Biotechnologies, Santa Cruz, USA), anti p-P38 (Cell Signaling, Charlottesville, USA), 9-E10 (A14-Santa Cruz Biotechnologies) and anti-Flag (Invitrogen, Carlsbad, USA).

**Preparation of cDNA and RT-PCR.** The total RNA of the cells was extracted using Trizol (Life Technologies, Carlsbad, USA) following the manufacturer's instructions. In each reaction 2 micrograms of total RNA were transcribed to cDNA using reverse transcriptase M-Mlv (Promega).

**Transfection and analysis of gene expression.** The 293T cells were transfected transiently using the calcium phosphate method, as described previously ³⁹. The total amount of DNA was maintained constant at 10 µg per million cells. The X-DC cells were transfected transiently by electroporation with 3 µg of pLNCX-GSE24-2 per million cells. The cells VA13 MEFs were transfected with 16 µg of DNA/per million cells using lipofectamine plus (Invitrogen), following the manufacturer's instructions. The protein extracts were prepared and luciferase measured using a commercial kit (Promega). Each test was carried out in triplicate and the experiments were repeated three times. The efficiency of transfection was corrected by co-transfection of the plasmid p-CMV-Renilla.

**Gel mobility shift assay.** The 293T cells were transfected with the plasmids pCDNA3-E1024-2 or empty vector and stable lines were generated through selection with G-418. These cells express the fusion protein with epitope E10-myc GSE24-2. The extraction of nuclear protein ⁴⁰ binding reactions were carried out as described previously ⁴¹. The oligonucleotide TEL1 containing the telomere sequence, and CXext which hybridises with the hanging zone of TEL1 (as negative control)⁴¹ were marked on end 5' with T4 polynucleotide kinase (New England Biolabs, Beverly, MA) and □³²P ATP (Amersham Bioscences, Orsay, France). GSE24-2 binding competition was carried out using an antibody against epitope 9E10. The complexes bound to DNA were separated in non-denaturing gels of acrylamide at 0.5% in Tris Borate EDTA buffer.

**Viability assays and number of accumulated duplications (PLD).** Cell growth and viability were determined using the crystal violet staining method as described previously ³⁹. The DKC fibroblasts were mixed after the infection and G-418-resistant cells were selected. Growth was measured in this culture quantifying the PLDs as of the first step of the cells following selection.

### Bibliographical references

1.- de Lange T. Protection of mammalian telomeres. Oncogene. 2002; 21: 532-540.
2.- Blackburn EH. Switching and signaling at the telomere. Cell. 2001; 106: 661-673.
3.- Collins K, Mitchell JR. Telomerase in the human organism. Oncogene. 2002; 21: 564-579.
4.- Colgin LM, Reddel RR. Telomere maintenance mechanisms and cellular immortalization. Curr Opin Genet Dev. 1999; 9: 97-103.
5.- Harley CB. Telomerase therapeutics for degenerative diseases. Curr Mol Med. 2005; 5: 205-211.
6.- Blasco MA. Telomeres and human disease: ageing, cancer and beyond. Nat Rev Genet. 2005; 6: 611-622.
7.- Cohen SB, Graham ME, Lovrec GO, et al, Protein composition of catalytically active human telomerase from immortal cells. Science, 2007; 315: 1850-1853
8.- Zhao J Q, Hoare S F, McFarlane R, et al. Cloning and characterization of human and mouse telomerase RNA gene promoter sequences. Oncogene. 1998; 16: 1345-1350.
9.- Takakura M, Kyo S, Kanaya T, et al. Cloning of human telomerase catalytic subunit (hTERT) gene promoter and identification of proximal core promoter sequences essential for transcriptional activation in immortalized and cancer cells. Cancer Res. 1999; 59: 551-5577.
10.- Gomez D, Aouali N, Renaud A, et al. Resistance to senescence induction and telomere shortening by a G-quadruplex ligand inhibitor of telomerase. Cancer Res. 2003; 63: 6149-6153.
11.- Li H, Xu D, Li J, Berndt MC, Liu JP. Transforming growth factor beta suppresses human telomerase reverse transcriptase (hTERT) by Smad3 interactions with c-Myc and the hTERT gene. J Biol Chem. 2006; 281: 25588-255600.
12.- Levens D, Duncan R C, Tomonaga T, et al. DNA conformation, topology, and the regulation of c-myc expression. Curr Top Microbiol Immunol. 1997; 224: 33-46.
13.- Siebenlist U, Hennighausen L, Battey J, Leder P. Chromatin structure and protein binding in the putative regulatory region of the c-myc gene in Burkitt lymphoma. Cell. 1984; 37: 381-391.
14.- Mirkin S M, Frank-Kamenetskii MD. H-DNA and related structures. Annu Rev Biophys Biomol Struct. 1994; 23: 541-576
15.- Siddiqui-Jain A, Grand CL, Bearss DJ, Hurley LH. Direct evidence for a G-quadruplex in a promoter region and its targeting with a small molecule to repress c-MYC transcription. Proc Natl Acad Sci U S A. 2002; 99: 11593-11598.
16.- Bessler M, Wilson DB, Mason PJ. Dyskeratosis congenita and telomerase. Curr Opin Pediatr. 2004; 16: 23-28.
17.- Marrone A, Mason PJ. Dyskeratosis congenita. Cell Mol Life Sci. 2003; 60: 507-517.
18.- Mason PJ, Wilson DB, Bessler M. Dyskeratosis congenita -- a disease of dysfunctional telomere maintenance. In Curr Mol Med. 2005; 5: 159-170.
19.- Heiss NS, Knight SW, VulliamyTJ, et al. X-linked dyskeratosis congenita is caused by mutations in a highly conserved gene with putative nucleolar functions. Nat Genet. 1998; 19: 32-38.
20.- Knight SW, Heiss NS, Vulliamy TJ, et al. X-linked dyskeratosis congenita is predominantly caused by missense mutations in the DKC1 gene. Am J Hum Genet. 1999; 65: 50-58.
21.- Mitchell J, Wood E, Collins K. A telomerase component is defective in the human disease dyskeratosis congenita. Nature. 1999; 402: 551-555.
22.- Normand C, Capeyrou R, Quevillon-Cheruel S, Mougin A, Henry Y, Caizergues-Ferrer M. Analysis of the binding of the N-terminal conserved domain of yeast Cbf5p to a box H/ACA snoRNA. RNA. 2006; 12: 1868-82.
23.- Henras A, Henry Y, Bousquet-Antonelli C, Noaillac-Depeyre J, Gélugne JP, Caizergues-Ferrer M. Nhp2p and Nop10p are essential for the function of H/ACA snoRNPs.; EMBO J. 1998; 17: 7078-7090.
24.- Pogacic V, Dragon F, Filipowicz W.Human H/ACA small nucleolar RNPs and telomerase share evolutionarily conserved proteins NHP2 and NOP10.. Mol Cell Biol. 2000; 20: 9028-40.
25.- Wang C, Meier UT. Architecture and assembly of mammalian H/ACA-small nucleolar and telomerase ribonucleoproteins. EMBO J. 2004; 23: 1857-1867
26.- Henras AK, Capeyrou R, Henry Y, et al Cbf5p, the putative pseudouridine synthase of H/ACA-type snoRNPs, can form a complex with Gar1p and Nop10p in absence of Nhp2p and box H/ACA snoRNAs. RNA. 2004; 10: 1704-12.
27.- Rashid R, Liang B, Baker D L, et al. Crystal structure of a Cbf5-Nop10-Gar1 complex and implications in RNA-guided pseudouridylation and dyskeratosis congenita.. Mol Cell.2006; 21: 249-260.
28.- Darzacq X, Kittur N, Roy S, et al Stepwise RNP assembly at the site of H/ACA RNA transcription in human cells. J Cell Biol. 2006; 173: 207-18.
29.- Tollervey D, Kiss T. Function and synthesis of small nucleolar RNAs. Curr Opin Cell Biol. 1997; 9: 337-342.
30.- Fu D, Collins K. Distinct biogenesis pathways for human telomerase RNA and H/ACA small nucleolar RNAs. Mol Cell. 2003; 11: 1361-1372.
31.- Wong JM, Collins K. Telomerase RNA level limits telomere maintenance in X-linked dyskeratosis congenita. Genes Dev. 2006; 2: 2848-2858.
32.- Oh S, Song YH, Kim UJ, Yim J, Kim TK. In vivo and in vitro analyses of Myc for differential promoter activities of the human telomerase (hTERT) gene in normal and tumor cells. Biochem Biophys Res Commun. 1999; 263: 361-365
33.- Kalinin A, Thomä NH, lakovenko A, Heinemann I, Rostkova E, Constantinescu AT, Alexandrov K. Expression of mammalian geranylgeranyltransferase type-II in Escherichia coli and its application for in vitro prenylation of Rab proteins. Protein Expr Purif. 2001; 22: 84-91.
34.- Sirinavin C, Trowbridge AA. Dyskeratosis congenita: clinical features and genetic aspects. Report of a family and review of the literature. J Med Genet. 1975; 12: 339-354
35.- Trowbridge AA, Sirinavin C, Linman JW. Dyskeratosis congenita: hematologic evaluation of a sibship and review of the literature. Am J Hematol. 1977; 3: 143-152.
36.- Roninson IB, Gudkov AV, Holzmayer TA, et al. Genetic suppressor elements: new tools for molecular oncology. Cancer Res. 1995; 55: 4023-4028.
37.- Wright WE, Shay JW, Piatyszek MA. Modifications of a telomeric repeat amplification protocol (TRAP) result in increased reliability, linearity and sensitivity. Nucleic Acids Res. 1995; 23: 3794-3795.
38.- Gomez D, Mergny JL, Riou JF. Detection of telomerase inhibitors based on g-quadruplex ligands by a modified telomeric repeat amplification protocol assay Cancer Res. 2002; 62: 3365-8.
39.- Sanchez-Perez I, Perona R. Lack of c-Jun activity increases survival to cisplatin. FEBS Lett. 1999; 453: 151-158.
40.- Perona R, Montaner S, Saniger L, Sanchez-Perez I, Bravo R, Lacal JC.1997 Activation of the nuclear factor-kappaB by Rho, CDC42, and Rac-1 proteins. Genes Dev. 1997; 11: 463-75.
41.- Gomez D, O'Donohue MF, Wenner T, Douarre C, Macadre J, Koebel P, Giraud-Panis MJ, Kaplan H, Kolkes A, Shin-ya K, Riou JF.The G-quadruplex ligand telomestatin inhibits POT1 binding to telomeric sequences in vitro and induces GFP-POT1 dissociation from telomeres in human cells. Cancer Res. 2006; 66: 6908-12.
42.- Redon S, Bombard S, Elizondo-Riojas MA, Chottard JC. Platination of the (T2G4) 4 telomeric sequence: a structural and cross-linking study. Biochemistry. 2001; 40: 8463-8470.
43.- Ishibashi T, Lippard SJ. Telomere loss in cells treated with cisplatin. Proc Natl Acad Sci U S A. 1998; 95: 4219-4223.
44.- Parkinson GN, Lee M P, Neidle S. Crystal structure of parallel quadruplexes from human telomeric DNA. Nature. 2002; 417: 876-880.
45.- Bednarek A, Shilkaitis A, Green A, et al. Suppression of cell proliferation and telomerase activity in 4-(hydroxyphenyl)retinamide-treated mammary tumors. Carcinogenesis. 1999; 20: 879-883.
46.- Kim JH, Kim JH, Lee GE, Kim SW, Chung IK. Identification of a quinoxaline derivative that is a potent telomerase inhibitor leading to cellular senescence of human cancer cells. Biochem J. 2003; 373: 523-529.
47.- Hurley LH. DNA and its associated processes as targets for cancer therapy. Nat Rev Cancer. 2002; 2: 188-200.
48.- Sun D, Thompson B, Cathers BE, et al. Inhibition of human telomerase by a G-quadruplex-interactive compound. J Med Chem. 1997; 40: 2113-2116.
49.- Cuesta J, Read MA, Neidle S. The design of G-quadruplex ligands as telomerase inhibitors. Mini Rev Med Chem. 2003; 3: 11-21.
50.- Chau NP, Deschatrette J, Wolfrom C Reversal of hepatoma cells resistance to anticancer drugs is correlated to cell proliferation kinetics, telomere length and telomerase activity. Anticancer Res. 2005; 25: 3279-85
51.- Lin CP, Liu JD, Chow JM, Liu CR, Liu HE. Small-molecule c-Myc inhibitor, 10058-F4, inhibits proliferation, downregulates human telomerase reverse transcriptase and enhances chemosensitivity in human hepatocellular carcinoma cells. Anticancer Drugs. 2007; 18: 161-70.
52.- Sun PM, Wei LH, Luo MY, Liu G, Wang JL, Mustea A, Könsgen D, Lichtenegger W, Sehouli J. The telomerase activity and expression of hTERT gene can serve as indicators in the anti-cancer treatment of human ovarian cancer. Eur J Obstet Gynecol Reprod Biol. 2007; 130: 249-57.
53.- Poole JC, Andrews LG, Tollefsbol TO. Activity, function, and gene regulation of the catalytic subunit of telomerase (hTERT). Gene. 2001; 269: 1-12.
54.- Meier UT, Blobel G. NAP57, a mammalian nucleolar protein with a putative homolog in yeast and bacteria.. J Cell Biol. 1994; 127: 1505-14.
55.- Meier UT. The many facets of H/ACA ribonucleoproteins. Chromosoma. 2005; 114: 1-14.
56.- Zucchini C, Strippoli P, Biolchi A, et al. The human TruB family of pseudouridine synthase genes, including the Dyskeratosis Congenita 1 gene and the novel member TRUB1. Int J Mol Med. 2003; 11: 697-704.
57.- Wu KJ, Grandori C, Amacker M, et al. Direct activation of TERT transcription by c-MYC. Nat Genet. 1999; 21: 220-224.
58.- Mochizuki Y, He J, Kulkarni S, Bessler M, Mason PJ. Mouse dyskerin mutations affect accumulation of telomerase RNA and small nucleolar RNA, telomerase activity, and ribosomal RNA processing. Proc Natl Acad Sci U S A. 2004; 101: 10756-10761.
59.- Bryan TM, Marusic L, Bacchetti S, Namba M, Reddel RR The telomere lengthening mechanism in telomerase-negative immortal human cells does not involve the telomerase RNA subunit. Hum Mol Genet. 1997; 6: 921-6.
60.- Kunifuji Y, Gotoh S, Abe T, Miura M, Karasaki Y. Down-regulation of telomerase activity by anticancer drugs in human ovarian cancer cells. Anticancer Drugs. 2002; 13: 595-598.
61.- Postel EH, Berberich SJ, Rooney JW, Kaetzel DM. Human NM23/nucleoside diphosphate kinase regulates gene expression through DNA binding to nuclease-hypersensitive transcriptional elements. J Bioenerg Biomembr. 2000; 32: 277-284.
62.-Nosaka K, Kawahara M, Masuda M, Satomi Y, Nishino H. Association of nucleoside diphosphate kinase nm23-H2 with human telomeres. Biochem Biophys Res Commun. 1998; 243: 342-348.

## Claims

1. Use of an inductor or activator compound GSE24.2 for producing a medicament or pharmaceutical composition for treating a preferably human illness or pathological situation, caused by a process of altered senescence.

2. Use of a compound according to claim 1 **characterised in that** the illness is due to a natural process of senescence as a result of natural ageing.

3. Use of a compound according to claim 1 **characterised in that** the illness is due to a pathological process of accelerated senescence.

4. Use of a compound according to claim 2 **characterised in that** the natural senescence process affects epithelial tissue or a tissue with a high level of proliferation.

5. Use of a compound according to claim 4 **characterised in that** the epithelial tissue belongs to the following group: skin, preferably, of the epidermis, gut, cornea, liver, lung and hair bulb.

6. Use of a compound according to claim 4 **characterised in that** the tissue with a high level of proliferation belongs to the following group: cells of the haematopoietic system, preferably lymphocytes, macrophages and erythrocytes; germinal cells,

7. Use of a compound according to claim 4 to 6 **characterised in that** the cells are somatic cells, trunk cells or embryonic cells.

8. Use of a compound according to claim 1 **characterised in that** the inductor agent GSE24.2 is a sequence of nucleotides that allows the expression of a protein or peptide that induces recovery from senescence inside the cells of a mammal, preferably human, and that is composed of one or several sequences of nucleotides GSE 24.2 belonging to the following group:
a) a sequence of nucleotides composed of a human GSE 24.2 nucleotides sequence (SEQ ID N01),
b) a variant of the nucleotide sequence of a) that shows an identity in the sequence of nucleotides of at least 90% with SEQ ID N01,
c) a fragment of any of the sequences of a) and b), and
d) a sequence of nucleotides, genetic construction, which comprises any sequence of a), b) and c).

9. Use of a compound according to claim 8 **characterised in that** the sequence of nucleotides is a sequence of DNA, cDNA or mRNA.

10. Use of a compound according to claim 8 **characterised in that** the sequence of nucleotides of sequence GSE 24.2 of a) is composed of SEQ ID N01.

11. Use of a compound according to claim 8 **characterised in that** the sequence of nucleotides of sequence GSE 24.2 of c) is composed of SEQ ID N03 or SEQ ID NO5, which encode peptide domains Trub I and Trub II, respectively.

12. Use of a compound according to claim 8 **characterised in that** the sequence of nucleotides is a genetic construction GSE 24.2 which comprises sequence GSE24.2.

13. Use of a compound according to claim 8 **characterised in that** the sequence of nucleotides is an expression vector GSE 24.2 comprising a sequence of nucleotides GSE 24.2 or a genetic construction GSE 24.2 and allows the expression of a protein or peptide capable of diminishing or reversing the process of senescence.

14. Use of a compound according to claim 13 **characterised in that** the expression vector is the plasmid pLNCX 24.2.

15. Use of a compound according to claim 1 **characterised in that** the inductor agent GSE24.2 is a protein or peptide presenting senescence reversal activity inside the cells of a mammal, preferably human, and comprising one or several sequences of amino acids belonging to the following group:
a) a sequence of amino acids composed of a sequence of human GSE 24.2 amino acids (SEQ ID N02),
b) a variant sequence of amino acids according to the sequence of a) which shows an identity in the amino acid sequence of at least 90% with the polypeptide of SEQ ID N02,
c) a fragment of any of the sequences of a) and b), and
d) a sequence of amino acids that comprises any sequence belonging to a), b) and c).

16. Use of a compound according to claim 15 **characterised in that** the inductor agent GSE24.2 is a protein whose sequence of amino acids of a) is composed of SEQ ID N02.

17. Use of a compound according to claim 15 **characterised in that** the inductor agent GSE24.2 is a protein whose sequence of amino acids of c) is composed of SEQ ID N04 or SEQ ID N06.

18. Use of a compound according to claim 1 **characterised in that** inductor agent GSE24.2 is a eukaryote cell, preferably a human cell, transformed by means of these nucleotide sequences GSE 24.2, of different cell lineages, which can be used as regenerator cells of human tissues.

19. Pharmaceutical composition or medicament for treating an illness or pathology related to alterations of senescence **characterised in that** it comprises a compound or activator compound GSE 24.2, in a therapeutically effective quantity together with, optionally, one or more adjuvants and/or pharmaceutically acceptable vehicles and that is capable of diminishing or reversing the process of senescence, and obtained by means of the use of the invention and obtained according to the use of a compound according to claims 1 to 18.

20. Use of the pharmaceutical composition according to claim 19 in a procedure of treating an illness or pathological situation caused by a process of altered senescence affecting human beings.

21. Use of the pharmaceutical composition according to claim 19 in a procedure of immortalising eukaryote cells, preferably human or of animals used in biomedical research.

22. Eukaryote cell, preferably a human cell, **characterised in that** it presents a reversed or diminished process of senescence and **in that** it comprises the element GSE24.2.


LISTADO DE SECUENCIAS

<110> CONSEJO SUPERIOR DE INVESTIGACIONES CIENTIFICAS,
      UNIVERSIDAD AUTÓNOMA DE MADRID

<120> USO DE AGENTES INDUCTORES GSE24.2 PARA LA ELABORACIÓN DE
      COMPOSICIONES FARMACÉUTICAS PARA EL TRATAMIENTO DE ENFEMEDADES
      QUE CURSAN CON SENESCENCIA CELULAR

<130> GSE 24.2

<160> 6

<170> PatentIn version 3.3

<210> 1
<211> 165
<212> DNA
<213> Homo sapiens

<220>
<221> CDS
<222> (1)..(165)
<223> Secuencia GSE 24.2

<400> 1
ggt ttc att aat ctt gac aag ccc tct aac ccc tct tcc cat gag gtg          48
Gly Phe Ile Asn Leu Asp Lys Pro Ser Asn Pro Ser Ser His Glu Val
1               5                   10                  15

gta gcc tgg att cga cgg ata ctt cgg gtg gag aag aca ggg cac agt          96
Val Ala Trp Ile Arg Arg Ile Leu Arg Val Glu Lys Thr Gly His Ser
            20                  25                  30

ggt act ctg gat ccc aag gtg act ggt tgt tta atc gtg tgc ata gaa          144
Gly Thr Leu Asp Pro Lys Val Thr Gly Cys Leu Ile Val Cys Ile Glu
        35                  40                  45

cga gcc act cgc ttg gtg aag                                              165
Arg Ala Thr Arg Leu Val Lys
    50                  55

<210> 2
<211> 55
<212> PRT
<213> Homo sapiens

<400> 2

Gly Phe Ile Asn Leu Asp Lys Pro Ser Asn Pro Ser Ser His Glu Val
1               5                   10                  15

Val Ala Trp Ile Arg Arg Ile Leu Arg Val Glu Lys Thr Gly His Ser
2           20                  25                  30

Gly Thr Leu Asp Pro Lys Val Thr Gly Cys Leu Ile Val Cys Ile Glu
        35                  40                  45

Arg Ala Thr Arg Leu Val Lys
    50                  55

<210> 3
<211> 54
<212> DNA

<213> Homo sapiens

<220>
<221> CDS
<222> (1)..(54)

<220>
<221> misc_feature
<222> (4)..(50)
<223> Dominio TrubI

<400> 3
ggt ttc att aat ctt gac aag ccc tct aac ccc tct tcc cat gag gtg      48
Gly Phe Ile Asn Leu Asp Lys Pro Ser Asn Pro Ser Ser His Glu Val
1               5                   10                  15

gta gcc                                                              54
Val Ala

<210> 4
<211> 18
<212> PRT
<213> Homo sapiens

<400> 4

Gly Phe Ile Asn Leu Asp Lys Pro Ser Asn Pro Ser Ser His Glu Val
1               5                   10                  15

Val Ala

<210> 5
<211> 75
<212> DNA
<213> Homo sapiens

<220>
<221> CDS
<222> (1)..(75)

<220>
<221> misc_feature
<222> (4)..(50)
<223> Dominio Trub II

<400> 5
cac agt ggt act ctg gat ccc aag gtg act ggt tgt tta atc gtg tgc      48
His Ser Gly Thr Leu Asp Pro Lys Val Thr Gly Cys Leu Ile Val Cys
1               5                   10                  15

ata gaa cga gcc act cgc ttg gtg aag                                  75
Ile Glu Arg Ala Thr Arg Leu Val Lys
            20                  25

<210> 6
<211> 25
<212> PRT
<213> Homo sapiens

<400> 6

His Ser Gly Thr Leu Asp Pro Lys Val Thr Gly Cys Leu Ile Val Cys
1               5                   10                  15
Ile Glu Arg Ala Thr Arg Leu Val Lys
            20                  25